# EUROPEAN PATENT APPLICATION

(11) **EP 3 805 759 A1**
(43) Date of publication of application: **14.04.2021**
(21) Application number: 20200476.8
(22) Date of filing: 07.10.2020
(51) Int. Cl.: G01N 33/566, G01N 33/49, A61K 35/14, C12N 5/078, G01N 33/574, C12N 15/115

(54) **METHOD FOR THE ISOLATION OF INTACT EXTRACELLULAR VESICLES**

(30) Priority: 08.10.2019 IT 201900018239
(71) Applicant: Consiglio Nazionale Delle Ricerche, 00185 Roma (IT)
(72) Inventor: CHIARI, Marcella, 20131 MILANO (IT)
(74) Representative: Zaccaro, Elisabetta

(57) **Abstract**

The present invention relates to a method for the isolation of intact extracellular vesicles from biological tissues and fluids.

Under a further aspect, the present invention relates intact extracellular vesicles, obtainable from the method herein described, and their use as a diagnostic and therapeutic tool.

## Description

### FIELD OF THE INVENTION

The present invention relates to a method for the isolation of intact extracellular vesicles from biological tissues and fluids.

Under a further aspect, the present invention relates to intact extracellular vesicles obtainable from the method herein described, and their use as a diagnostic and therapeutic tool.

### STATE OF THE ART

Cells release in the extracellular environment vesicles that are attracting considerable interest in the scientific community due to their role in intercellular communication. Extracellular vesicles (EVs) is a generic term for all secreted vesicles. They can be further classified in subpopulations including: exosomes, (30-150 nm), microvesicles (MVs) (100-350 nm) and apoptotic bodies (500-1000 nm) according to their cellular origin. Due to their key role in cellular functions, EVs are of great interest in basic research as well as in future diagnostic applications or clinical settings.

Isolation of EVs, particularly exosomes, from complex biological fluids whilst preserving their physical characteristics is critical for downstream applications. To date, different exosome isolation techniques have been developed, they include: differential ultracentrifugation, size-based techniques, precipitation and immunoaffinity capture-based techniques.

The majority of purification protocols exploit the physical properties of these vesicles to extract them from heterogeneous, biological samples. The most widely applied method, differential ultracentrifugation, comprises a series of high speed spins (∼100,000 × g) to selectively sediment bio-nanoparticles from solution. Ultracentrifugation in a density gradient provides preparations free from contaminating cellular debris, but this method is not suited for high-throughput applications.

Ultrafiltration or size exclusion chromatography exploit size difference for the isolation. Ultrafiltration is faster than ultracentrifugation but the use of centrifuge force may result in the deformation and breaking up of large vesicles potentially altering the results of downstream analysis. Size exclusion chromatography (SEC) has little risk for damage or aggregation of EVs as the technique relies on gravity flow. However, while SEC purifies EVs, it also dilutes them. Therefore, the method is not suitable for harvesting EVs from cell culture media, where they are often less concentrated than in plasma.

Several commercial kits, such as the Invitrogen Total Exosome Isolation Kit (Life Technologies, USA) and ExoSpin Exosome Purification Kit (Cell Guidance Systems, USA), add poly-ethylene glycol or similar substances to promote sedimentation of exosomes from solution during low speed centrifugation (10,000-20,000 × g). However, with these methods the presence of contaminating cellular and protein debris has been noted.

In general, a significant problem in EVs isolation is that both, blood and cell culture media, contain large numbers of nanoparticles (some non-vesicular) in the same size range as exosomes. For example, Wang et al. ((2010) 'Export of microRNAs and microRNA-protective protein by mammalian cells', Nucleic Acids Research, 38(20), pp. 7248-7259) found that large number of miRNAs are contained within extracellular protein complexes rather than exosomes. Therefore, separation by physical characteristics is not possible.

To overcome this problem, methods that exploit immunoaffinity interactions between proteins and receptors on the membrane of exosomes and their antibodies have been proposed (Jørgensen, M. et al. (2013) 'Extracellular Vesicle (EV) Array: microarray capturing of exosomes and other extracellular vesicles for multiplexed phenotyping', Journal of Extracellular Vesicles, 2(1), p. 20920). The presence of proteins and receptors in the membrane of exosomes offers an excellent opportunity to exploit immunoaffinity interactions between those proteins (antigens) and their antibodies providing highly specific techniques for the isolation of Evs. Different immunoaffinity capture-based techniques have been developed for the isolation of exosomes. Ideally, exosome biomarkers for immuno isolation are solely expressed or highly concentrated on the surface of exosomes from specific biological sources.

Isolation of Evs may be carried out by an approach that allows their recovery as intact vesicles by applying an immunoaffinity capture where the antibody are immobilized by the so called DNA directed immobilization (DDI) strategy (Niemeyer CM et al (1999) "DNA-directed immobilization: efficient, reversible, and site-selective surface binding of proteins by means of covalent DNA-streptavidin conjugates." Anal Biochem 268:54-63). DDI was first introduced in the fabrication of protein microarray in order to study biological events involving proteins and peptides (R. Wacker, C.M. Niemeyer, DDI-µFIA-a readily configurable microarray-fluorescence immunoassay based on DNA-directed immobilization of proteins, ChemBioChem. 5 (2004) 453-459).

The problem that arises in a conventional immunoaffinity approach is that EVs are tightly bound to antibodies directed against antigen proteins on their surface. Releasing vesicles requires strong acidic buffers and/or detergents, both conditions that damage the nanoparticles and impair certain types of downstream analysis.

The need and importance is increasingly felt for the development of a method for the isolation of EVs that does not have the drawbacks that have been encountered when using the techniques of ultracentrifugation, SEC and immunoaffinity.

It is therefore object of the present invention the development of a method for EV isolation, which allows the purification from biological tissues and fluids in an efficient way, without damaging the EVs.

### SUMMARY OF THE INVENTION

The present invention concerns a method for the isolation of intact extracellular vesicles (EVs), in particular exosomes, from a biological tissue or fluid comprising the steps of:
a. functionalizing a support with a single strand oligonucleotide;
b. providing a ligand having a tag which is complimentary to the single strand oligonucleotide of the functionalized support of step a.;
c. incubating the functionalized support of step a. with a ligand such as an antibody, a peptide or an aptamer having a tag of step b. in order to obtain an immobilized ligand;
d. incubating the immobilized ligand of step c. with a biological fluid to allow the capture of the EVs through the binding of said immobilized ligand with the EVs contained in said biological fluid and to obtain a substrate with captured EVs;
e. incubating the captured EVs of step d. with a restriction enzyme;
f. isolating the EVs;
   wherein said isolated EVs of step f. are intact.

Discussed herein are methods for rapid isolation of EVs by an approach that allows their recovery as intact vesicles. The method applies an immunoaffinity or an affinity capture approach to collect exosomes on the surface of a substrate combined with an enzymatic approach for their release. The method proposed is enabled by the strategy used to immobilize antibodies directed against surface antigens or affinity ligands that is based on the so called DNA directed immobilization (DDI) strategy (Niemeyer CM, Boldt L, et al (1999) DNA-directed immobilization: efficient, reversible, and site-selective surface binding of proteins by means of covalent DNA-streptavidin conjugates. Anal Biochem 268:54-63). DDI was first introduced into the fabrication of protein microarray in order to study biological events involving proteins and peptides (R. Wacker et al. DDI-µFIA-a readily configurable microarray-fluorescence immunoassay based on DNA-directed immobilization of proteins, ChemBioChem. 5 (2004) 453-459).

In a second aspect, the present disclosure relates to an intact extracellular vesicle (EV), having a size in the range from 30nm to 350nm, preferably from 30nm to 150 nm and from 100nm to 350 nm according to their cellular origin and obtainable from the method according to the present invention.

In a still further aspect, the present disclosure relates to the use of the intact extracellular vesicle obtainable from the method according to the present invention, as a diagnostic tool in liquid biopsy.

In a still further embodiment, the invention provides for the use of the intact extracellular vesicle obtainable from the method according to the present invention as a diagnostic tool in liquid biopsy for the analysis of microRNA, DNA, protein cargo, microarray, ELISA, flow cytometry with different detection schemes including fluorescence, interferometry, surface plasmon resonance.

In a still further aspect, the present disclosure relates to the use of the intact extracellular vesicle obtainable from the method according to the present invention, as a therapeutic tool.

Further applications are on-chip EVs analysis (microarray format with different detection schemes including fluorescence, interferometry, surface plasmon resonance), EVs isolation from various sources including complex biological samples (e.g. serum, plasma, urine), and the conjugation on platforms for integrated isolation and analysis.

As it will be further described in the detailed description of the invention, the problem underlying the present invention is that of making available a method for isolating intact EVs, which are not contaminated by cellular and protein debris and are ready for further downstream analysis.

### BRIEF DESCRIPTION OF THE DRAWINGS

The characteristics and advantages of the present invention will be apparent from the detailed description reported below, from the Examples given for illustrative and non-limiting purposes, and from the annexed Figures 1-6, wherein:
Figure 1: shows a real-time IRIS analysis of mass accumulation of DNA-directed antibodies. a) results for ssDNA-AntiCD9. b) results for ssDNA-AntiCD63, as described in Example 2.
Figure 2: shows a schematic representation of the invention as described in Example 3.
Figure 3. Nanoparticle Tracking Analysis of samples recovered using DDI approach, covalently functionalized magnetic beads (CIC) and ultracentrifugation (UC), as described in Example 5.
Figure 4. Nanoparticle Tracking Analysis of sample recovered using DDI approach (light grey curve) and DNAse I containing buffer (dark grey curve), as described in Example 5.
Figure 5. Western Blot analysis of unpurified Plasma, samples separated by DDI approach, Conventional immunocapturing and ultracentrifugation, as described in Example 5.
Figure 6. Particles captured on a microarray chip, detected using SP-IRIS. a) particles captured from EV containing sample. b) particles captured using DNAse I buffer, as described in Example 5.

### DETAILED DESCRIPTION OF THE INVENTION

The present invention concerns a method for the isolation of intact extracellular vesicles (EVs) from a biological tissue or fluid comprising the steps of:
a. functionalizing a support with a single strand oligonucleotide;
b. providing a ligand such as an antibody, a peptide or an aptamer having a tag which is complimentary to the single strand oligo nucleotide of the functionalized support of step a.;
c. incubating the functionalized support of step a. with the ligand having a tag of step b. in order to obtain an immobilized antibody;
d. incubating the immobilized ligand of step c. with a biological fluid to allow the capture of the EVs through the binding of said immobilized antibody with the EVs contained in said biological fluid and to obtain a substrate with captured EVs;
e. incubating the captured EVs of step d. with a restriction enzyme;
f. isolating the EVs;
   wherein said isolated EVs of step f. are intact.

The present invention discloses an innovative approach for reversible capturing of exosomes on their surface. In order to release intact vesicles, the so-called DNA directed immobilization (DDI) strategy for the functionalization of magnetic beads was applied to the EV isolation.

In a conventional immunoaffinity approach EVs are tightly bound to antibodies directed against antigen proteins on their surface. Releasing vesicles requires strong acidic buffers and/or detergents, both conditions that damage the nanoparticles and impair certain types of downstream analysis.

Surprisingly in the method of the present invention the issues related to protein immobilization and availability were overcome, and in addition to these features, intact EVs are obtained thanks to the reversible character of the DNA-linker. This linker can be denaturated (changing temperature or ionic strength of the medium) or cleaved using enzymes, causing the release of the target-probe complex from the surface in mild conditions.

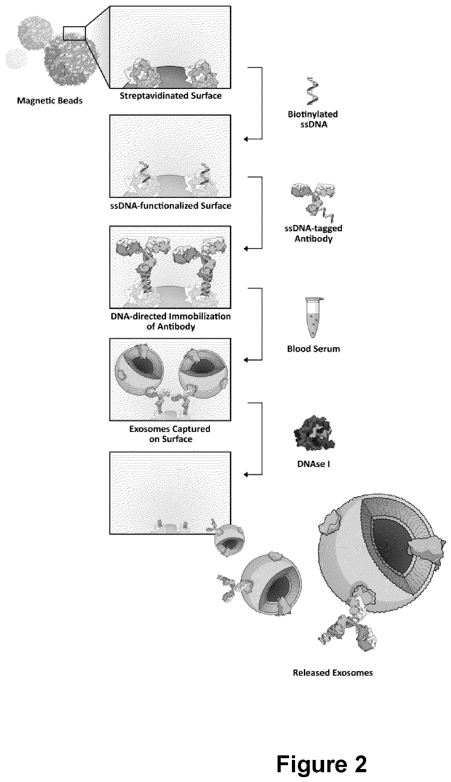

A schematic representation of the invention is depicted in Figure 2. A ligand such as an antibody is encoded by a specific DNA sequence covalently attached to it (to the antibody or to the ligand) and binds to its complementary ssDNA probes on the surface of micro-particles (or to any solid substrate) via sequence specific DNA-DNA hybridization. DDI takes advantage of surface-bound capture oligonucleotides to selectively bind proteins tagged with complementary oligonuclotides.

Several methods can be used to conjugate the ligand (in general an antibody, a protein or a peptide) with DNA. DNA and protein conjugates have a variety of applications and thus numerous methods of conjugation are under development. It has been proven that DNA linkers are excellent spacers that enhance the availability of binding sites for analyte capture by decreasing the steric hindrance. This feature has been recently exploited for the capturing of viruses in an interferometric technique called single particle interforemetric reflectance imaging sensor (SP-IRIS). Besides increasing capturing efficiency, DDI allows facile release of EVs as the double stranded DNA linker can be cleaved by an enzymatic reaction catalyzed by DNAse.

Different exosomes sub-populations can be captured specifically using antibodies or capture probes tagged with different oligonucleotides that are complementary to sequences immobilized on the surface.

The term "ligand" as used herein refers to a molecule or more generally to a compound which is capable of binding to EVs. The ligand of interest may bind specifically to a protein (antigen) on the surface of EVs or non specifically to the EV membrane. It includes antibodies, peptides and aptamers

The term "exosome" as used herein is meant to include a small secreted vesicle or "EV" (typically about 30-150 nm) which may contain, or have present in its membrane, a nucleic acid, protein, or other biomolecules and may serve as a carrier of this cargo between diverse locations in a body or biological system. The term "biological fluid", as used herein, means any fluid isolated or derived from an organism including prokaryotes, eukaryotes, bacteria, fungi, yeast, invertebrates, vertebrates, reptiles, fish, insects, plants and animals. Media taken from cultured cells ("conditioned media", cell media, cell culture media) may be a biological fluid.

According to one aspect, the described invention provides a method for the isolation of intact EVs, wherein said support is chosen from the group consisting of: a magnetic bead, a membrane, a cell culture plate, a test tube, a slide, a plain or nanostructured surface, a microplate, a tube, a microchannel, pillars, a disk-like piece, particles (beads), and the like.

In a preferred aspect, said support is functionalized with by covalent bonding or by biotinilation. In another embodiment said support is functionalized with a polymer bearing chemical functional groups able to react with functionalities introduced at the end of the oligonucleotide.

Exosomes may be isolated from a variety of biological sources including mammals such as mice, rats, guinea pigs, rabbits, dogs, cats, bovine, horses goats, sheep, primates or humans. Typically, exosomes are isolated from biological fluids such as serum, plasma, whole blood, urine, saliva, breast milk, tears, sweat, joint fluid, cerebrospinal fluid, semen, vaginal fluid, ascetic fluid and amniotic fluid. Exosomes may also be isolated from experimental samples such as media taken from cultured cells ("conditioned media", cell media, cell culture media).

According to a further aspect, in the method for the isolation of intact EVs of the described invention, said biological fluid is chosen from the group consisting of plasma, blood serum, cell culture medium, urine, saliva, tears, whole blood, breast milk, sweat, joint fluid, cerebrospinal fluid, semen, vaginal fluid, ascetic fluid and amniotic fluid.

EVs may also be isolated from tissue samples such as surgical samples, biopsy samples, tissues, feces, plant tissue, insect tissue, and cultured cells. When isolating EVs from tissue sources it may be necessary to homogenize the tissue in order to obtain a single cell suspension followed by lysis of the cells to release the EVs. When isolating EVs from tissue samples it is important to select homogenization and lysis procedures that do not result in disruption of the EVs.

EVs may be isolated from freshly collected samples or from samples that have been stored frozen or refrigerated. Although not necessary, higher purity EVs may be obtained if fluid samples are clarified before purification, to remove any debris from the sample. Methods of clarification include centrifugation, ultracentrifugation, filtration or ultrafiltration.

Any surface protein can be targeted to immunopurify EVs, (for instance CD9, CD63, CD81, CD82), MHC class I and II, HSP70, Annexin V, Flotillin and EpCAM. In the following examples anti tetraspanin antibodies were used as an example.

The EVs can be purified using microparticles or collected on the surface of a microarray slides or a microtiter plate. When microparticles are used, they can be separated from the bulk fluid using either magnetic field or centrifugal force.

Under a still preferred aspect, the restriction enzyme of step e. of the described method is a DNAse, preferably DNAse I.

Surprisingly it has been found that the method of the present invention, which relies on the use of the DDI method applied to the capture of EVs has allowed for the isolation of intact vesicles by the use of a DNAse to release the EVs. Conventional methods to release EVs based on the disruption of the antibody antigen binding cause severe damages to EVs as they require the use of buffers with low pHs. Also, methods based on DNA denaturation, that require changes of physical properties (e.g. heating and sonication) or chemical conditions (such as alkaline treatment, formamide and DMSO) are not compatible with the stability of EVs. For this reason we propose to release EVs by cutting the DNA linker enzymatically with DNAse I.

In a further preferred aspect, in the method for EV isolation of the present invention there is an additional washing step between steps d. and step e.. Preferably, said washing step is carried out with a buffer.

According to a further aspect, the described invention provides a method for the isolation of intact EVs, wherein said antibody is an antibody directed against exosome-specific tetraspanins, preferably (CD9, CD63, CD81, CD82), MHC class I and II, HSP70, Annexin V, Flotillin and EpCAM.

According to a still further aspect, the described invention provides a method for the isolation of intact EVs, wherein said antibody is an antibody directed against exosome-specific surface proteins, preferably said exosome-specific surface proteins are MHC class I and II, HSP70, Annexin V, Flotillin and EpCAM. Antibodies directed against EVs-specific surface proteins, such as, for instance, tetraspanins, preferably CD9 and CD63, were functionalized with DNA and used as probes both in particle based extraction system or in microarray analytical platforms. DNA modified antibodies were captured by hybridization with complementary sequences immobilized through interaction between biotin and streptavidine in the case of magnetic particles or through covalent bonds between a terminal amino group on the oligonucleotide and a succinimidyl esther on a polymer-coated microarray surface.

In a second embodiment, the present disclosure relates to an intact extracellular vesicle (EV) obtainable from the method according to the present invention. Preferably the intact isolated EV has a size in the range from 30nm to 150 nm and from 100 nm to 350 nm according to their cellular origin.

In a further embodiment, the present disclosure relates to the use of the intact extracellular vesicle obtainable from the method according to the present invention, as a diagnostic tool in liquid biopsy, preferably for the analysis of microRNA, DNA or protein cargo.

In a further embodiment, the present disclosure relates to the use of the intact extracellular vesicle obtainable from the method according to the present invention, as a diagnostic tool in microarray, ELISA, flow cytometry (just to mention some examples format) with different detection schemes including fluorescence, interferometry, surface plasmon resonance), or as therapeutic tools.

In a further embodiment, the present disclosure relates to the use of the intact extracellular vesicle obtainable from the method according to the present invention, as a therapeutic tool.

Various embodiments and aspects of the present invention as delineated hereinabove and as claimed in the claims section below find experimental support in the following examples.

### EXAMPLES

### Example 1.: Antibody functionalization with ssDNA

Antibodies directed against exosome-specific tetraspanins (CD9 and CD63) were modified with a linker bearing N-oxysuccinimide ester at one end and dibenzocyclooctine (DBCO) at the other end to allow their binding to azido modified oligonucleotides as reported below.

**1.1 Synthesis of ssDNA-AntiCD63:** 4.4 µL of a 4 mM PBS solution of DBCO-NHS ester were added to 200 µL of sodium azide-free mouse anti-human CD63 IgG (0.70 mg/ml) and the solution was allowed to react for 30 min at room temperature. The reaction was quenched by adding 20 µL of 1M TRIS-HCI, pH=8.0, and the unreacted ester was removed, through centrifugation over an Amicon Ultra 10K filter (3 min at 12,000xg). The eluted solution was discarded and the protein was recovered in 200 µL of PBS. The oligonucleotide ssDNA-TAG-63 (8.2 µL, 100 µM) was added to 200 µL of DBCO-modified antibody (0.66 mg/ml) and the strain-promoted 1,3-dipolar dycloaddition of cycloalkynes to azide reaction was carried out overnight at 4°C. The unreacted oligonucleotide was eliminated through centrifugation over an Amicon Ultra 30K filter by centrifugation for 2 min at 12,000xg. After the final purification, 200 µL of ssDNA-modified AntiCD63 (0.47 mg/ml) were obtained. The antibody concentration was determined before each step using a Bradford assay. The sample (3 µL) was added to a solution of 2.4 mL of water and 0.6 mL of Bradford Reagent. The absorbance at 595 nm was measured using a Jasco V-530 UV/VIS Spectrophotometer.

**1.2 Synthesis of ssDNA-AntiCD9:** an identical procedure was used to tag CD9: 3.8 µL of DBCO-NHS ester 4 mM were added to 200 µL of sodium azide-free Mouse Anti-Human CD9 IgG (0.61 mg/ml) and the solution was allowed to react for 30 min at room temperature. Once the reaction was quenched and the unreacted ester removed, 7.4 µL of ssDNA-TAG-9 100 µM were added to 200 µL of DBCO-modified antibody (0.59 mg/ml) and the SPAAC reaction was carried out overnight at 4°C. After final purification, 200 µL of ssDNA-modified AntiCD9 (0.42 mg/ml) were obtained.

### Example 2.: DNA-Directed Immobilization (DDI) of antibodies on silicon chips.

**2.1 Probe spotting on the surface of microarray:** both antibody and oligonucleotide solutions were prepared. Amino-modified oligonucleotides complementary to the antibody tag (ssDNA-PROBES) were dissolved at different concentrations (0.5, 5, 10 and 25 µM) in a solution of 150 mM sodium phosphate buffer containing 0.01% sucrose monolaurate at pH 8.5. The probes were then spotted onto the surface of different silicon/silicon oxide chips, depending on the detection technique used. In particular, oxide layers of 110 nm and 55 nm were use for IRIS and SP-IRIS measurements respectively. All chips were coated with copoly (DMA-NAS-MAPS), a polymer commercially available with the trade name of MCP-2 (Lucidant Polymers Inc., Sunnyvale CA, USA) using a noncontact microarray spotter (sciFLEXARRAYER S12, Scienion, Berlin) equipped with a 80 µm nozzle. MCP-2 is a ter-copolymer of N,N-dymethilacrylamide (DMA) (97% in moles), N-acryloyloxysuccinimide (NAS) (2% in moles) and 3-(trimethoxysilyl)propyl methacrylate (MAPS) (1% in moles), which forms a 3-D layer for the immobilization of antibodies with retained capture efficiency and suppression of non-specific binding.

Four hundred picoliters (pL) of solution were spotted at room temperature and 65% humidity. Immediately after spotting, the chips were stored overnight in a sealed chamber filled at the bottom with sodium chloride saturated water (40 g/100 mL H₂O). After incubation, the chips were treated with a blocking solution of ethanolamine (50 mM in 0.1M Tris/HCI buffer pH 9), at room temperature for 1 h. Then chips were rinsed with bidistilled water and dried under nitrogen stream.

### 2.2 Evaluation of DNA-Directed Immobilization efficiency with real-time IRIS measurements:

In order to assess the effectiveness of the method a set of microarray tests was devised and the experimental parameters optimized.

First, the antibody immobilization density was evaluated with a real-time interferometric technique called interferometric reflectance Imaging Sensor (IRIS). Different concentrations of ssDNA-CD9 and ssDNA-CD63, antiCD9, antiCD63 and COCU8 (a sequence of DNA used as negative control) were spotted on a silicon chip. Different concentrations of ssDNA-PROBE-9 and ssDNA-PROBE-63 (0.5, 5, 10 and 25 µM) AntiCD9, AntiCD63 and a 10 µM solution of ssDNA (COCU8 10) that does not bind the ssDNA-tagged antibodies, were deposited on a silicon chip, which was assembled with an adhesive spacer and an AR coated glass slide, to form a chamber for in-flow measurements. The cartridge thus obtained was loaded into the Interferometric Reflectance Imaging Sensor (IRIS) instrument and PBS was flowed for 15 min to stabilize the probes. A solution of ssDNA-AntiCD9 and ssDNA-AntiCD63, each at a concentration of 0.5 µg/mL, was then injected into the system and recirculated for 20 min at an average flow-rate of 200 µL/min. PBS was then flushed for 20 min at the same flow-rate as the washing buffer. The same procedure was repeated twice with different antibodies concentrations (i.e. 5 µg/mL and 10 µg/mL).

Interferometric images were acquired during the whole process with a blue LED light (465 nm). Four-colour images, used to generate a look-up table for the conversion to mass density were acquired at the beginning and at the end of each step.

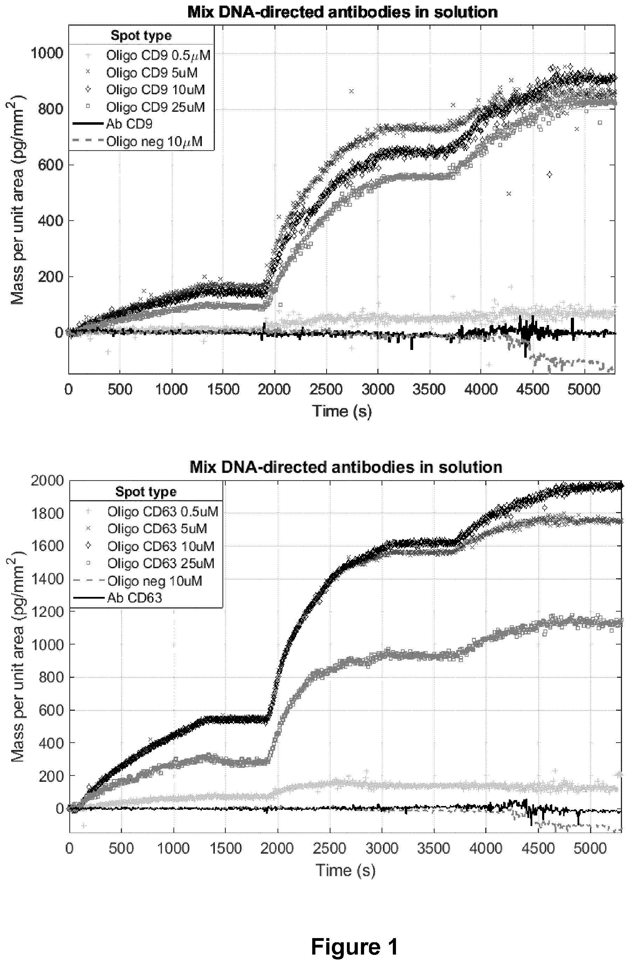

The results in terms of density of immobilized antibody are shown in Figure 1. As expected, no mass is accumulated on the negative control (COCU8) and on antibodies immobilized through conventional amine coupling, while the mass density increases on the spots of ssDNA-probes, confirming the success of DDI. The amount of antibody captured depends on the density of ssDNA-PROBE which in turn depends on the concentration of spotted probe. At low concentration (0.5 µM) only a small amount of antibody is captured, reaching saturation with 5 µg/mL of ssDNA-antibody while at 5 and 10 µM concentration a greater amount of DNA-modified antibody is immobilized. Interestingly, the density of captured antibody decreases when the probe is spotted at 25 µM concentration probably due to crowding effect. Moreover for both antibodies, the highest concentration of ssDNA-antibodies tested (10 µg/mL) produces noisy signals. For this reason it was decided to use the intermediate concentration (5 µg/mL) in the following experiments. In the purification experiment, ssDNA-AntiCD63 was used since it provided a higher immobilization yield.

### Example 3.: Extracellular Vesicle purification using DNA-Directed Immobilization of AntiCD63 on magnetic beads (Figure 2).

**3.1 Microparticles functionalization:** DNA tagged antibodies were immobilized on the surface of magnetic beads and used to immunocapture Evs. Prior to their use, streptavidin-coated magnetic beads (Dynabeads M-270 Streptavidin, Invitrogen) were washed three times with Binding and Washing buffer (B&W) (5 mM Tris-HCI, pH 7,5; 0,5 mM EDTA; 1 M NaCI) according to the manufacturer's protocol. Then 500 µg of beads were added to 100 µL of 1 µM biotinylated ssDNA-Probe-63 solution. The suspension was stirred for 30 min at 23°C, then the solution was removed and the beads were washed twice with B&W buffer, then once with PBS.

Oligonucleotide modified beads (500 µg) were incubated with 100 µL of ssDNA-AntiCD63 antibody (160 µg/mL) for 1h at 25°C, then the solution was removed and the beads were washed twice in PBS.

**3.2 Capture and release of EVs from magnetic beads:** DNA-directed ssDNA-AntiCD63 functionalized magnetic beads (500 µg) were incubated with 250 µL of human plasma obtained from blood samples of healthy blood donors.

After incubation, the excess of plasma was discharged and the beads were incubated with 50 µL of 250 mKunitz/µL solution of DNAse I from bovine pancreas in 10 mM Tris/HCI, pH 7.5 buffer, containing 5 mM MgCl2 and 130 µM CaCI2. At the end of the incubation, performed at 37°C for 1 h, the beads were separated using a magnet and the buffer was recovered and used in subsequent analysis.

### Example 4.: Extracellular Vesicles purification using conventional immunocapturing

**4.1 Magnetic beads for conventional immunocapturing (CIC):** Carboxylated magnetic beads (Abraxys Inc., Warminster, PA, USA) were covalently functionalized with AntiCD63 (clone MEM259, HansaBioMed, Estonia) via conventional amine coupling. Ten µL of 2.5 mg/mL functionalized magnetic beads (0.5 µm in diameter) were added to 500 µL of plasma and incubated for 4 h at 25°C and 1400 rpm. Then the beads were washed twice with 1 mL of PBS. To release EVs, 10 µL of elution buffer (HansaBioMed, Tallinn, Estonia) was added to the beads and the solution was vortexed for 30 sec. After 5 min, the solution was vortexed again for 30 sec and finally 40 µL of PBS were added.
**4.2 Ultracentrifugation:** Plasma sample was centrifuged at 10000 x g for 10 min to remove debris and aggregates. The supernatant was collected, diluted 1:2 with PBS and filtered through 0.22 µm syringe filters (Corning, NY, USA) to remove contaminating apoptotic bodies, and cell debris. The clarified medium was then centrifuged at 150.000 x g at 4°C for 90 min (Beckman Coulter TL-100 Ultracentrifuge, TLA-100.3 fixed angle rotor) to sediment extracellular vesicles. The supernatant was carefully removed, and the pellet was resuspended in PBS and stored at
   -20°C until used.

### Example 5.: Characterization of released extracellular vesicles

**5.1 Nano Tracking Analysis:** All samples were analyzed using Nanosight NS300 (Malvern Panalytical, Malvern, UK). Videos were analyzed by the in-build NanoSight Software NTA 3.2 Dev Build 3.2.16. The Camera type, Camera level, and Detect Treshold were sCMOS, 14 and 4, respectively. The number of completed tracks in NTA measurments was 5 (a 60 seconds movie was registered for each measurement). All samples were diluted in PBS to a final volume of 1 mL. The ideal concentration was assessed by pre-testing the optimal particle per frame value (20-100 particles per frame).

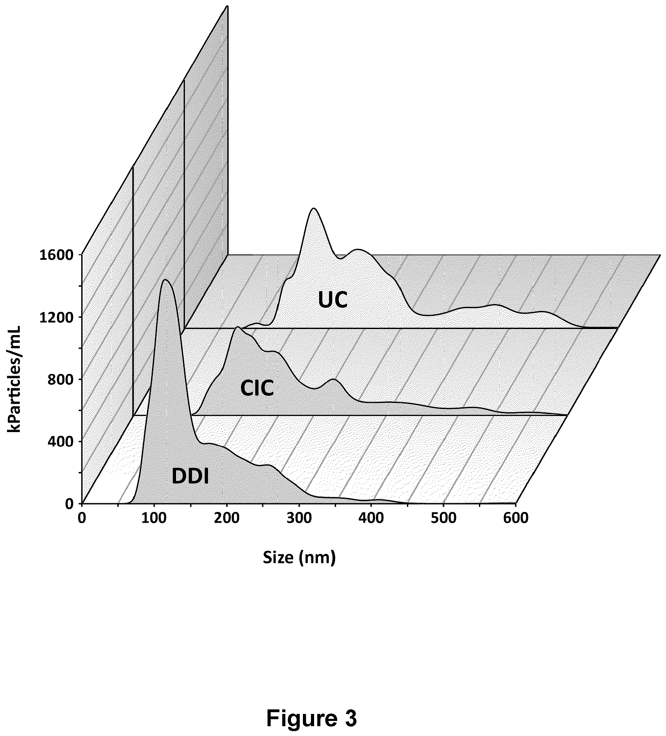

The size distributions of the three samples determined by NTA are reported in Figure 3. Only the sample purified by immunoaffinity with DNA directed immobilized antibodies has the typical size distribution of EVs ranging between 100 and 200 while the profile of the other two samples looks different and shows the presence of large particle contaminants (400-500 nm). The profile of the sample immunocaptured by conventional approach was somehow expected, since the release buffer contains a detergent that causes membrane degradation. Less obvious was the profile of the sample obtained by ultragentrifugation that, in principle should isolate intact vesicles.

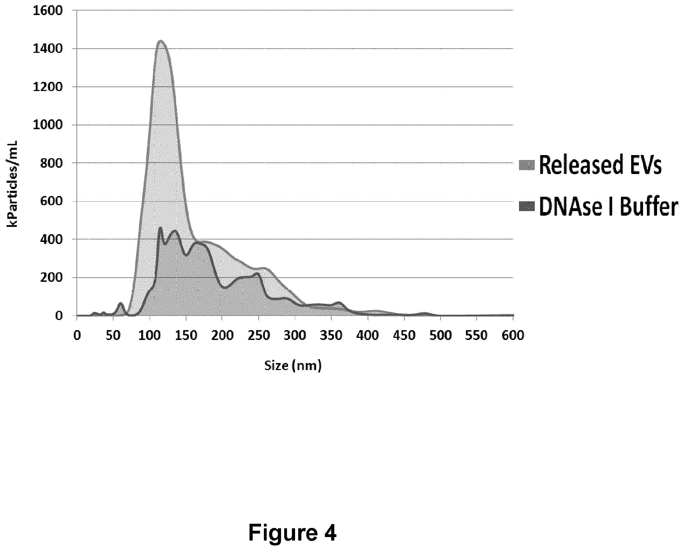

In Figure 4 the NTA profile of nanoparticles released from magnetic beads with DNAse I (light grey curve) was compared with that of the buffer containing only the enzyme (dark grey curve). It is evident from the traces of Figure 4 that the particles in the 75-200 nm range come from the beads, while an important percentage of bigger particles (200-300 nm) are present in the enzyme buffer.

**5.2 Western Blot Analysis (WB):** Unpurified plasma, vesicles purified using either by DDI approach, conventional immunocapturing or ultracentrifugation were analyzed by Western Blot. In particular, the content of membrane proteins (CD9, CD63 and CD81) and cargo protein TSG101 were determined, while calnexin was used as negative control. Protein concentration in unpurified plasma and purified samples was determined using DC™ protein assay kit (BioRad, CA, USA). Bovine serum albumin (BSA) was used as standard. For tetraspanins detection, samples were lysed in non reducing sample buffer (0.25 M Tris-HCI pH 6.8, 40% glycerol, 8% SDS, and 0.04% bromophenol blue) and boiled for 5 min at 95°C. Then, 15 µg of proteins were loaded on 12% SDS-PAGE gel. For other proteins, detection samples were lysed in reducing sample buffer (0.25 M Tris-HCI pH 6.8, 40% glycerol, 8% SDS, 5% 2-mercaptoethanol and 0.04% bromophenol blue) and boiled for 5 min at 95°C. The 24 µg of proteins were loaded on 8% SDS-PAGE gel. After protein separation, the gels were electro-transferred onto a nitrocellulose membrane. Nonspecific binding sites were blocked with 5% (w/v) skimmed milk in T-TBS (150mM NaCl, 20mM Tris-HCI pH 7.4, and 0.5% Tween 20). The different transferred membranes were incubated overnight at 4°C with the following antibodies: anti-CD9 (1:1000, #555370, BD Pharmingen, CA, USA), anti-CD63 (1:1000, #556019, BD Pharmingen, CA, USA), anti-TSG101(1:800, #NB200-112, Novus Bio, CO, USA) and anti-Calnexin (1:1000, #C7617, Sigma-Aldrich, MO, USA). After several washes in T-TBS, the membranes were incubated with goat anti-mouse IgG conjugated to horse-radish peroxidase (1:5000, #170-6516, BioRad Laboratories Inc., CA, USA) for 45 min. Positive immunoreactive bands were detected by the enhanced chemiluminescence method (ImmobilonTM HRP substrate, #WBKLS0500, Millipore Corp., MA, USA).

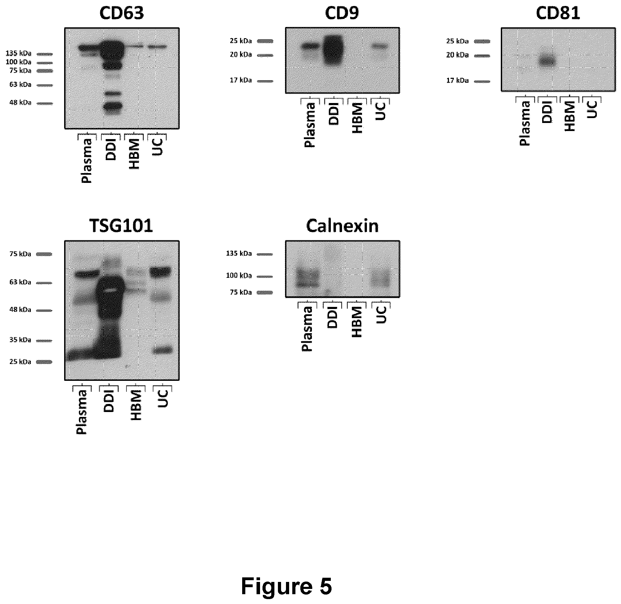

The WB results shown in Figure 5 clearly demonstrate that EV-related proteins are present in samples separated by the DDI method in larger amount compared to the other methods and this is more prominent since the result was obtained considering a lower plasma volume, 2-3 times less, with respect to the other methods. In addition, DDI method allowed a recover of a higher amount of total proteins, which derived from EVs, as shown in Figure 5 but also that they are particularly enriched in comparison with plasma sample. Interestingly, despite the abundance of EV-related proteins, no signal was registered for calnexin, proving that the method described herein provides highly pure samples of extracellular vesicles.

Conventional immunocapturing approach and ultracentrifugation produce low intensity or no signals for typical EV proteins in comparison to the proposed approach in the loaded amount of samples (CD9 and CD81 are not detected using conventional beads and CD81 is missing in ultracentrifugation sample). The results of Western blot demonstrate the superior enrichment in EVs of the sample separated with DDI approach. As prior reported, ultracentrifugation, although still considered the gold standard for EVs purification, showed a high signal for calnexin, suggesting that presence of contaminants in the preparation.

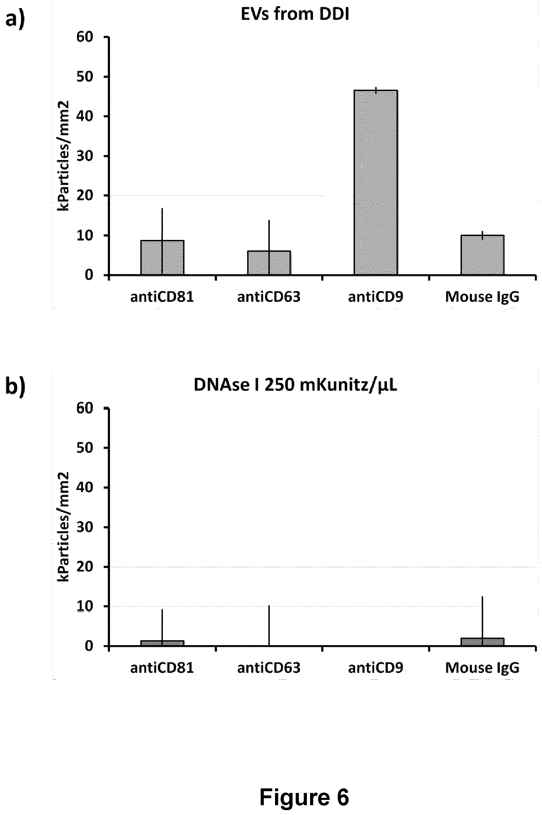

**5.3 SP-IRIS analysis.** To confirm that the sample solution contained nanoparticles with size and shape corresponding to EVs released vescicles were characterized by SP-IRIS instrument using the commercial microarray silicon chips (ExoView Tetraspanin Kit, NanoView Biosciences, USA). The incubation was carried out for 2.5 hours at room temperature, and then chips were washed for 10 min in the washing buffer of the kit, rinsed with bidistilled water and dried. Finally, chips were scanned using SP-IRIS technology. The Single-Particle Interferometric Reflectance Imaging Sensor (SP-IRIS) takes advantage of an optical configuration that is similar to the classic IRIS one; in this case, however, the resolution is much higher, due to the presence of a high-magnification objective, and the application is different: the SP-IRIS indeed was conceived to achieve the digital sizing and counting of individual nanoparticles bound to the surface, down to a diameter of 50nm. A single wavelength LED is used, which shall be carefully chosen to match the oxide layer thickness in order to achieve the best possible enhancement of the visibility of the nanoparticles. In our case, 60nm-Si/SiO2 commercial silicon chips, functionalized with antibodies directed against tetraspanins CD9, CD63 and CD81 and a mouse IgG as negative control (ExoView Tetraspanin Kit, Nanoview Biosciences, USA) were used in combination with a near-UV wavelength LED. Scans of the surface along the focal direction - that is, the direction parallel to the objective - were performed, and changes in contrast of a single nanoparticle at distinct focal planes can be directly correlated to its size. The chips were incubated with EVs purified on DDI magnetic beads, while a control chip was incubated with DNAse I buffer. The results are shown in Figure 6. The DNAse I buffer does not contain particles that can be captured by the antibodies on the chip. On the contrary, a significant amount of nanoparticles were captured on the anti-CD9 spot incubated with purified EVs. The low number of particles captured on CD81 spots can be explained by the low level of CD81 present in this preparation also confirmed by Western blotting while the low level of particles captured on antiCD63 spots might depend on the fact that most of the CD63 molecules on the surface are masked by the antibody used for their capture during the purification step. The antibody released by cleavage of the DNA linker could have interacted with the antigen, hampering the capture on the microarray surface.

### Materials and Methods

Dibenzocyclooctyne-N-hydroxysuccinimidyl ester (DBCO-NHS ester), phosphate buffer saline tablets (PBS), Tris, 37% chloric acid (HCI), sodium phosphate (Na3PO4), sucrose monolaurate, sodium chloride (NaCI), magnesium chloride (MgCl2), calcium chloride (CaCl2), Tween 20, glycerol, sodium dodecyl sulfate (SDS), bromophenol blue, Cy3-labeled rabbit anti-mouse IgG, Cy3-labeled streptavidin, DNAse I from bovine pancreas, IgGs from Mouse serum, ethanolamine, trehalose dihydrate and Amicon Ultra centrifugal filters (MWCO 10K and 30K) were purchased from Sigma Aldrich (St. Louis, MO, USA). Goat polyclonal anti-Alpha-lactalbumin antibody was obtained from GeneTex (Irvine, CA, USA). All solvents were used as received. Sodium azide free IgG antibodies: mouse anti-human CD9 (clone MEM-61) and mouse anti-human CD63 (clone MEM-259) were provided by Hansa BioMed Life Sciences Ltd (Tallinn, Estonia). Oligonucleotides with the following sequences were synthesized by MWG-Biotech AG (Ebevsberg, Germany): ssDNA-TAG-63:
5'-AAAAAGCCTACGAATGAACAGACTG-3' (SEQ ID NO:1), ssDNA-PROBE-63: 5'-ATATGTACCCACCGCATTCTCAGTCTGTTCATTCGTAGGC-3' (SEQ ID NO:2), ssDNA-TAG-9: 5'AAAAATACAGAGTTAGTCGCAGTGG-3' (SEQ ID NO:3), ssDNA-PROBE-9: 5'-ATCCGACCTTGACATCTCTACCACTGCGACTAACTCTGTA-3' (SEQ ID NO:4), COCU8: 5'-GCCCACCTATAAGGTAAAAGTGA-3' (SEQ ID NO:5). ssDNA-TAGs were modified with a C6-azido linker at the 5' end, while ssDNA-PROBEs and COCU8 were modified with a C6 amino or byotin linker at 5' end. These oligonucleotides were freeze-dried and resuspended in DI water at a final concentration of 100 µM before use. NV10B silicon chips were supplied by NanoView Biosciences (Boston, MA, USA).

Spotting is performed using SciFLEXARRAYER S12 (Scienion, Berlin, Germany).

Bradford protein quantification analysis was performed using a Jasco V-530 UV/VIS Spectrophotometer and data were analyzed using Spectra Manager software 1.54 (Jasco, MD, USA).

Centrifugation was carried out using Eppendorf MiniSpin (Eppendorf, Hamburg, Germany).

The efficiency of the DNA directed immobilization of antibodies was tested by real-time IRIS device, using a low-magnification IRIS setup designed to perform in-liquid measurements. The instrument is fully described in (Needham, J., Lortlar Ünlü, N., Ünlü, M. S., 2019. Interferometric Reflectance Imaging Sensor (IRIS) for Molecular Kinetics with a Low-Cost, Disposable Fluidic Cartridge. Chapter 2 in Biomimetic Sensors in the Springer Methods in Molecular Biology series, Vol. 2027). A custom-made MATLAB software was used to convert the raw signal data to mass density. The analysis is illustrated in detail in (Sevenler D., Ünlü M.S., 2016. Numerical techniques for high-throughput reflectance interference biosensing. Journal of Modern Optics, 63, 1115-1120).

Interferometric analysis of EVs count and size were performed exploiting SP-IRIS technique using ExoView™ R100 for image acquisition and nanoViewer 2.6.0 software for analysis (NanoView Biosciences Inc., MA, USA). Nano Tracking Analysis was performed with NanoSight NS300 using 3.2 Dev Build 3.2.16 software (Malvern Instruments Ltd, Malvern, United Kingdom).

From the above description and the above-noted examples, the advantage attained by the product described and obtained according to the present invention are apparent.

## Claims

1. Method for the isolation of intact extracellular vesicles (EVs) from a biological tissue or fluid comprising the steps of:
a. functionalizing a support with a single strand oligonucleotide;
b. providing a ligand having a tag which is complimentary to the single strand oligonucleotide of the functionalized support of step a.;
c. incubating the functionalized support of step a. with the ligand having a tag of step b. in order to obtain an immobilized ligand;
d. incubating the immobilized ligand of step c. with a biological fluid to allow the capture of the EVs through the binding of said immobilized ligand with the EVs contained in said biological fluid and to obtain a substrate of captured EVs;
e. incubating the captured EVs of step d. with a restriction enzyme;
f. isolating the EVs;
wherein said isolated EVs of step f. are intact.

2. The method according to claim 1, wherein said ligand is chosen from the group consisting of: an antibody, a peptide or an aptamer.

3. The method according to anyone of claims 1 or 2, wherein said support is chosen from the group consisting of: a magnetic bead, beads, a membrane, a cell culture plate, a test tube, a slide, a microplate, a microchannel, a pillar or a disk-like piece.

4. The method according to anyone of claims 1 to 3, wherein said support is functionalized with the ligand by covalent bonding or by biotinilation.

5. The method according to anyone of claims 1 to 4, wherein said biological fluid is chosen from the group consisting of plasma, blood serum, cell culture medium, urine, saliva, tears, whole blood, breast milk, sweat, joint fluid, cerebrospinal fluid, semen, vaginal fluid, ascetic fluid and amniotic fluid.

6. The method according to anyone of claims 1 to 5, wherein said restriction enzyme is a DNAse, preferably DNAse I.

7. The method according to anyone of claims 1 to 6, having an additional washing step between steps d. and step e..

8. The method according to claim 7, wherein said washing step is carried out with a buffer.

9. The method according to anyone of claims 1 to 8, wherein said antibody is an antibody directed against exosome-specific surface proteins.

10. The method according to anyone of claims 1 to 9, wherein said antibody is an antibody directed against exosome-specific tetraspanins, preferably CD9, CD63, CD81, CD82.

11. The method according to anyone of claims 1 to 9, wherein said antibody is an antibody directed against exosome-specific surface proteins MHC class I and II, HSP70, Annexin V, Flotillin and EpCAM.


SEQUENCE LISTING

<110> CONSIGLIO NAZIONALE DELLE RICERCHE

<120> METHOD FOR THE ISOLATION OF INTACT EXTRACELLULAR VESICLES

<130> P021481EP-01

<150> IT102019000018239
<151> 2019-10-08

<160> 5

<170> BiSSAP 1.3.6

<210> 1
<211> 25
<212> DNA
<213> Artificial Sequence

<220>
<223> ssDNA-TAG-63

<400> 1
aaaaagccta cgaatgaaca gactg                                          25

<210> 2
<211> 40
<212> DNA
<213> Artificial Sequence

<220>
<223> ssDNA-PROBE-63

<400> 2
atatgtaccc accgcattct cagtctgttc attcgtaggc                          40

<210> 3
<211> 25
<212> DNA
<213> Artificial Sequence

<220>
<223> ssDNA-TAG-9

<400> 3
aaaaatacag agttagtcgc agtgg                                          25

<210> 4
<211> 40
<212> DNA
<213> Artificial Sequence

<220>
<223> ssDNA-PROBE-9

<400> 4
atccgacctt gacatctcta ccactgcgac taactctgta                    40

<210> 5
<211> 23
<212> DNA
<213> Artificial Sequence

<220>
<223> COCU8

<400> 5
gcccacctat aaggtaaaag tga                                      23